# EUROPEAN PATENT APPLICATION

(11) **EP 4 810 035 A1**
(43) Date of publication of application: **23.09.2026**
(21) Application number: 24890783.4
(22) Date of filing: 14.11.2024
(51) Int. Cl.: A61K 31/407, A61K 9/20, A61K 9/48, A61P 17/06

(54) **TREATMENT OF MODERATE TO SEVERE PLAQUE PSORIASIS**

(30) Priority: 14.11.2023 CN 202311518258
(71) Applicant: Ganzhou Hemay Pharmaceutical, Co., Ltd, Jiangxi Province (CN)
(72) Inventor: ZHANG, Hesheng, Ganzhou, Jiangxi 341600 (CN); LI, Xingwen, Ganzhou, Jiangxi 341600 (CN); CHANG, Yukun, Ganzhou, Jiangxi 341600 (CN); ZHANG, Donglei, Ganzhou, Jiangxi 341600 (CN)
(74) Representative: J A Kemp LLP
(86) International application number: PCT/CN2024/132112
(87) International publication number: WO 2025/103428

(57) **Abstract**

Disclosed are a compound of Formula (I), a stereoisomer thereof, or a pharmaceutically acceptable salt thereof for use in treating moderate to severe plaque psoriasis,

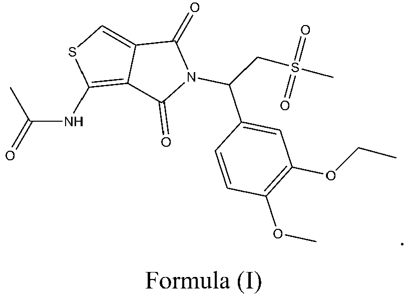

## Description

### REFERENCE TO RELATED APPLICATION

The present disclosure claims the full benefits of the Chinese patent application No. 202311518258.6, filed on November 14, 2023 with the National Intellectual Property Administration of the People's Republic of China, entitled "Moderate to severe plaque psoriasis treating", which is incorporated herein by reference in its entirety.

### FIELD

The present disclosure relates generally to the field of medicine, and more particularly, to the treatment of moderate to severe plaque psoriasis.

### BACKGROUND

Psoriasis is a chronic inflammatory skin disease triggered by environmental factors, influenced by multiple genes, and driven by an abnormal immune response. It typically manifests as scaly erythema or scaly plaques, which may be localized to one area or widely distributed over the entire body..

In addition to skin symptoms, psoriasis patients often have other systemic diseases, such as psychological diseases. Diseases that are significantly associated with psoriasis are currently referred to as psoriasis comorbidities. Systematic analysis based on large samples showed that among the comorbidities of psoriasis, the prevalence of anxiety was 30.2% (21.7% to 38.8%) and the prevalence of depression was 21.7% (15.1% to 28.3%).

The prevalence of anxiety and depression in psoriasis patients increases. The stress and shame due to the effects of skin lesions at exposed sites may cause depression and anxiety symptoms. Psychiatric depression may also exacerbate psoriasis through common inflammatory pathways. Vascular inflammation and coronary plaque burden were significantly increased in psoriasis patients with depression.

Psoriasis has no definitive treatment and all treatments should be effective in terms of improving quality of life and should be safe for long-term use. At the same time, controlling the complications associated with psoriasis, reducing the incidence of comorbidities, improving the physiological, psychological and social functions of patients, and improving the quality of life are also therapeutic goals as pursued.

Psoriasis patients often have immunodeficiency or immune dysregulation, which increases the risk of infection by pathogenic bacteria. Psoriasis patients need to take medicines. The long-term application of medicines such as immunosuppressants in existing psoriasis treatment medicines may lead to further reduction of immunity of psoriasis patients, thereby further increasing the risk of infection of pathogenic bacteria. Among these pathogens, *Mycobacterium tuberculosis* is a common one during treatment. About one third of the world's population is infected with *Mycobacterium tuberculosis* and 90% of the infected people may carry pathogenic bacteria for a long time to become patients with latent tuberculosis infection. Latent tuberculosis infection is a hotbed of active tuberculosis. The developmental outcome of patients with latent tuberculosis infection is highly uncertain and is a hidden minefield in the field of tuberculosis control. The immune function of patients with latent tuberculosis infection is decreased, which easily leads to the progression from latent to active tuberculosis.

The requirements for safety in the principle of treatment of psoriasis are very prominent. In the course of psoriasis treatment, tuberculosis infection and the like must be periodically tested prior to and/or during the use of methotrexate, glucocorticoids and biological agents. The risk of tuberculosis incidence is increased after treatment with biological agents. Following safe and effective treatment principles, attention is paid to a specific patient population with latent tuberculosis infection, a medical history of pulmonary tuberculosis infection/tuberculosis/tuberculous pleurisy in the treatment of psoriasis, appropriate treatment regimens are provided for patients with moderate to severe plaque psoriasis and psoriasis patients with latent tuberculosis infection or a medical history of tuberculosis, adverse reactions and potential risks of developing active tuberculosis are reduced, and it is of profound significance and impact to seek greater benefit for patients.

### SUMMARY

In one aspect, the present disclosure relates to a method for treating moderate to severe plaque psoriasis, comprising administering to a subject in need thereof a therapeutically effective amount of a compound of Formula (I), a stereoisomer thereof, or a pharmaceutically acceptable salt thereof, 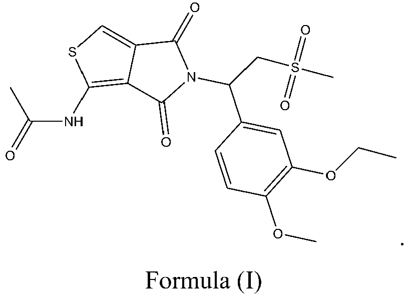 In another aspect, the present disclosure relates to a compound of Formula (I), a stereoisomer thereof, or a pharmaceutically acceptable salt thereof for use in treating moderate to severe plaque psoriasis in a subject, 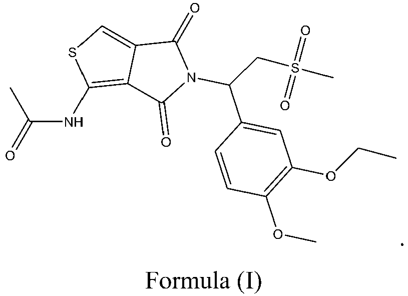 In a further aspect, the present disclosure relates to use of a compound of Formula (I), a stereoisomer thereof, or a pharmaceutically acceptable salt thereof in the manufacture of a medicament for treatment of moderate to severe plaque psoriasis in a subject, 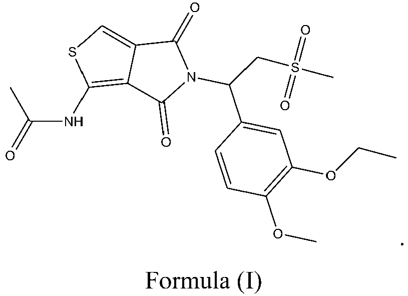 In yet another aspect, the present disclosure relates to a pharmaceutical composition for treating moderate to severe plaque psoriasis in a subject, comprising a compound of Formula (I), a stereoisomer thereof, or a pharmaceutically acceptable salt thereof, and a pharmaceutically acceptable carrier, diluent, or excipient,

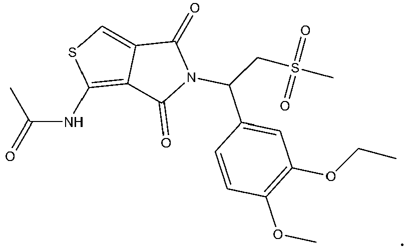

### DETAILED DESCRIPTION

In the following description, certain specific details are shown to provide a thorough understanding for various disclosed embodiments. One skilled in the relevant art, however, will recognize that the embodiments can be practiced without one or more of these specific details, or with other methods, components and materials, etc.

Unless the context required otherwise, throughout the specification and claims which follow, the term "comprise" and variations thereof, such as "comprises" and "comprising" are to be construed in an open, inclusive sense, namely "include, but not limited to".

As used in this disclosure and the appended claims, the singular reference without an indication of quantity includes the plural reference unless the context clearly dictates otherwise.

Reference throughout this specification to "one embodiment" or "an embodiment" or "in another embodiment", or "in some embodiments" means that a particular referent feature structure or characteristic described in connection with the embodiments is included in at least one embodiment. Therefore, the appearance of the phrases "in one embodiment", "in the embodiment", "in another embodiment", or "in some embodiments" in various places throughout this specification are not necessarily all referring to the same embodiment. Moreover, the particular features structures or characteristics can be combined in any suitable manner in one or more embodiments.

It should be noted that, as used in this specification and the appended claims, the singular forms "a", "an" and "the" comprise plural referents unless the context clearly states otherwise. Thus, for example, reference to a pharmaceutical composition comprising an "excipient" includes one excipient, or two or more excipients.

### Definition

Therefore, unless otherwise indicated, the following terms used in the specification and the appended claims have the following meanings:
In the present disclosure, the term "psoriasis" refers to a skin disorder stimulated by environmental factors, genetically controlled by multiple genes and immunologically mediated, which typically manifests as scaly erythema or plaque and is limited to one or a wide distribution throughout the body.

In the present disclosure, the term "plaque psoriasis" refers to a clinical classification of psoriasis, the main symptoms of which are dark red plaques or infiltrating erythema, overlying white, silvery white scales, which may have wax drop phenomenon, thin film phenomenon or petechial hemorrhage phenomenon. In the present disclosure, the term "moderate to severe plaque psoriasis" is a grade of psoriasis severity. The three-part method: the psoriasis is generally divided into three grades according to BSA, PASI and DLQI. The grading criteria are shown in Table 1.

**Table 1. Clinical grading standard of psoriasis severity**

| Mild | Moderate | Severe |
|---|---|---|
| Disease does not change the quality of life of patients. | Disease changes quality of life. | Disease seriously affects quality of life of patients. |
| The patients can minimize the impact of the disease without requiring treatment. | Patients expect treatment to improve quality of life. | It is ineffective against treatment with minimal adverse reactions. |
| Therapeutic measures do not have known adverse reactions (such as topical glucocorticoids) | Treatment measures have minimal adverse effects (although inconvenient, expensive, time-consuming and ineffective, patients consider that they have no impact on their recent and long-term health status.) | Patients desire treatment to improve the quality of life. Patients desire to receive treatment that has adverse effects on life state to alleviate or cure the disease. |
| BSA<3%, PASI<3 points, DLQI<6 points | BSA3%~<10%, PASI3-<10 points, DLQI6-<10 points | BSA≥10%, PASI ≥10 points, DLQI≥ 10 points one disease site (e.g., face, hands and feet, nails, genitals) |
| | | - arthropathy/arthritis |

| | | |
|---|---|---|
| Note: BSA: Body Surface Area; PASI: Psoriasis Area and Severity Index; DLQI: Dermatology Life Quality Index. | | |

The above classification index is defined as follows:
1. PASI is the most commonly used index for clinically evaluating the severity of psoriasis and is also widely used for evaluating the efficacy of treatment. The PASI score is an important index for evaluating the severity of psoriasis and is an index for quantifying the severity of psoriasis, including the skin lesion area score and the skin lesion severity score. The disease state of psoriasis is reflected by specific numbers, and the higher the score, the larger the lesion range and the more severe the lesions.
2. BSA: The area of a patient's single palm and the flexor surface of the fingers is defined as 1% of the total body surface area. The total sum of the patient's skin lesions is assessed by the number of palm areas, recorded as the BSA.
3. PGA: The score is determined based on the overall condition of erythema, scales and plaque induration of a patient. Erythema is scored from 0 to 5 as follows: none (hyperpigmentation may be present), slight, pink, moderate red, bright red and dusky red, respectively. Scaling is scored from 0 to 5 as follows: none, minimal fine scales, mild and mainly fine scales, moderate and coarse scales, severe and non-tenacious scales and very severe and very thick tenacious scales, respectively. Induration is scored from 0 to 5 as follows: no elevation, minimal elevation, mild elevation, moderate elevation, severe elevation and very severe elevation, respectively. The PGA score is calculated by dividing the sum of the above three scores by three.
   sPGA: Static Physician Global Assessment, used to determine the overall severity of a patient's psoriasis at a specific time point. The assessment reflects the overall damage of induration, erythema and scaling based on the scoring criteria, with specific numerical values representing the severity of the lesions.
4. DLQI: Assesses the impact of the disease on the patient's quality of life based on their subjective experience over the past week. The DLQI is a simple, concise and practical questionnaire used in clinical research to evaluate the effects of skin diseases. It consists of 10 items related to the subject's skin condition. The total DLQI score is the sum of all individual items, ranging from 0 to 30, where 30 represents the worst possible quality of life and 0 represents the best possible quality of life.

In the present disclosure, the term "HADS" refers to the Hospital Anxiety and Depression Scale, which is designed for screening anxiety and depression in patients within general hospital settings. The questionnaire is simple, concise and practical, and has been extensively studied in general medical environments. It consists of 7 items related to anxiety (A) and 7 items related to depression (D). All items are scored on a 4-point scale (0 to 3). The total score for anxiety or depression is the sum of the scores of the respective items, ranging from 0 to 21. Specifically, a score of 0 to 7 indicates no anxiety or depression, 8 to 10 indicates mild anxiety or depression, 11 to 14 indicates moderate anxiety or depression and 15 to 21 indicates severe anxiety or depression.

In the present disclosure, the term "biological agent" refers to a TNF-α inhibitor (including but not limited to etanercept, infliximab, adalimumab, certolizumab pegol), an IL-12/23 inhibitor (including but not limited to ustekinumab), an IL-17 inhibitor (including but not limited to secukinumab, ixekizumab, brodalumab), an IL-23 (p19 subunit) inhibitor (including but not limited to guselkumab, tildrakizumab), an IL-36 receptor inhibitor (including but not limited to spesolimab), and the like, which are currently used in the treatment of psoriasis.

In the present disclosure, the term "latent tuberculosis infection (LTBI)" means that the body is infected with *Mycobacterium tuberculosis*, but there is no clinical tuberculosis, no clinical symptoms, and no evidence of bacteriological and radiographic active tuberculosis. Diagnostic criteria for latent tuberculosis: subjects who test positive in *Mycobacterium tuberculosis*-specific T-cell interferon-gamma release assay (T-SPOT) and present no active tuberculosis clinical signs.

In the present disclosure, adverse events occurring in clinical trials are categorized using two common classification methods. The first method categorizes adverse reactions as mild, moderate or severe. Mild: Typically transient and generally does not interfere with the subject's activities of daily living; may require only minimal treatment. Moderate: The subject experiences discomfort, and activities of daily living are affected; treatment is usually required for relief, but there is no risk of significant or permanent harm to the subject. Severe: Causes significant impact on the subject's activities of daily living or severely affects clinical status; requires intensive treatment and intervention. The second method refers to the Common Terminology Criteria for Adverse Events (CTCAE), grading events from Grade 1 to Grade 5: Grade 1 (Mild): Asymptomatic or mild symptoms; clinical or diagnostic observations only; intervention not indicated. Grade 2 (Moderate): Minimal, local, or non-invasive intervention indicated; limiting age-appropriate instrumental Activities of Daily Living (ADL). Instrumental ADL refers to preparing meals, shopping for groceries or clothes, using the telephone, managing money, and the like. Self-care ADL refers to bathing, dressing and undressing, feeding self, using the toilet, taking medications, and not being bedridden. Grade 3 (Severe or medically significant but not immediately life-threatening): Hospitalization or prolongation of hospitalization indicated; disabling; limiting self-care ADL. Self-care ADL refers to bathing, dressing and undressing, feeding self, using the toilet, taking medications, and not being bedridden. Grade 4 (Life-threatening): Urgent intervention indicated. Grade 5 (Death): Death related to the adverse event.

In the present disclosure, the term "activation" refers to the transition of a patient's *Mycobacterium tuberculosis* infection status from latent tuberculosis infection to active tuberculosis infection; that is, the patient's infection status becomes active and the patient is in a state of active tuberculosis disease, requiring proactive and standardized treatment. In the present disclosure, the term "titrated dosing" refers to the process of gradually reaching the desired drug levels by starting with a low dose of the medication.

In the present disclosure, the term "compound of Formula (I)" refers to N-[5-[1-(3-ethoxy-4-methoxyphenyl)-2-(methylsulfonyl)ethyl]-4,6-dioxo-5,6-dihydro-5H-t hieno[3,4-c]pyrrol-1-yl]acetamide, the structural formula of which is as follows:

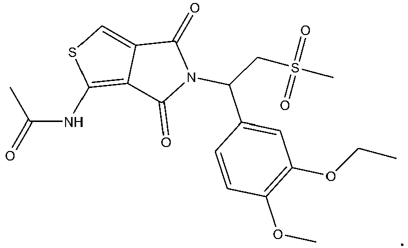

In the present disclosure, the term "a stereoisomer of compound of Formula (I)" refers to (S)-N-[5-[1-(3-ethoxy-4-methoxyphenyl)-2-(methylsulfonyl)ethyl]-4,6-dioxo-5,6-dihydro-5H-thieno[3,4-c]pyrrol-1-yl]acetamide, the structural formula of which is as follows:

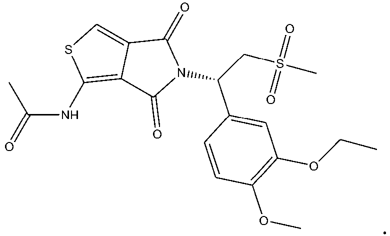

As used herein, the term "pharmaceutically acceptable" as used herein means the carrier, vehicle, diluent, excipient and/or salt must be compatible with the other ingredients of the formulation, and not deleterious to the recipient thereof.

As used herein, the term "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isosmotic agent, solvent, or emulsifier, etc, which has been approved by the United States Food and Drug Administration as being acceptable for use in humans or animals and have no adverse effects on preparing a pharmaceutical composition.

As used herein, the term "carrier" defines a compound that facilitates the incorporation of a crystal form of a compound into cells or tissues. For example, dimethylsulfoxide (DMSO) is generally used as a carrier, as it facilitates the uptake of many organic compounds into cells or tissues of an organism.

In the present disclosure, the term "pharmaceutically acceptable salts" include both "pharmaceutically acceptable acid addition salts" and "pharmaceutically acceptable base addition salts".

In the present disclosure, the term "pharmaceutically acceptable acid addition salt" refers to those salts which retain the biological effectiveness and properties of the free bases, which are not biologically or otherwise undesirable, and which are formed with inorganic acids such as, but not limited to hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecyl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleinic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid and the like.

In the present disclosure, the term "pharmaceutically acceptable base addition salt" refers to those salts which retain the biological effectiveness and properties of the free acids, which are not biologically or otherwise undesirable. These salts are prepared from addition of an inorganic or an organic base to the free acid. Salts derived from inorganic bases include, but are not limited, to sodium, potassium, lithium, ammonium, calcium, magnesium, iron, zinc, copper, manganese, aluminium salts and the like. In some embodiments, inorganic salts are the ammonium, sodium, potassium, calcium, and magnesium salts. Salts derived from organic bases include, but are not limited to, salts of primary, secondary and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucosamine, theobromine, triethanolamine, trometamol, purine, piperazine, piperidine, N-ethyl piperidine, polyamine resins and the like. In some embodiments, organic bases are isopropylamine, diethylamine, ethanolamine, trimethylamine, dicyclohexylamine, choline and caffeine.

As used herein, the term "mammal" means animals including, for example, dogs, cats, cows, sheep, horses, and humans. In some embodiments, mammals include humans.

As used herein, the term "pharmaceutical composition" refers to a formulation of the compound of Formula (I) or a pharmaceutically acceptable salt thereof in the present disclosure and a medium generally acceptable in the art for the delivery of the biologically active compound to mammals, e.g., humans. Such a medium includes all pharmaceutically acceptable carriers, diluents or excipients therefor.

As used herein, the term "therapeutically effective amount" refers to an amount of the compound or combination of the compounds that ameliorates, attenuates or eliminates a particular disease or condition or a symptom of a particular disease or condition, or prevents or delays the onset of a particular disease or condition or a symptom of a particular disease or condition. The amount of the compound of Formula (I) of the present disclosure which constitutes a "therapeutically effective amount" will vary depending on the compound, the condition and its severity, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, such as a human, having the disease or disorder of interest, and includes:
(i) preventing the disease or condition from occurring in a mammal, in particular, when such mammal is predisposed to the condition but has not yet been diagnosed as having it;
(ii) inhibiting the disease or condition, i.e., arresting its development; or
(iii) relieving the disease or condition, i.e., causing regression of the disease or condition.

In the present disclosure, the term "unit dose" refers to a dose for single use. For example, in the case of a tablet, the unit dose refers to the dose of a single tablet.

The compounds of the present disclosure or their pharmaceutically acceptable salts may contain one or more asymmetric centers and may thus give rise to enantiomers, diastereoisomers, and other stereoisomeric forms that may be defined, in terms of absolute stereochemistry, as (R)- or (S)-, or (D)- or (L)- for amino acids. The present disclosure is meant to include all such possible isomers, as well as their racemic and optically pure forms. Optically active (+) and (-), (R)- and (S)-, or (D)- and (L)-isomers may be prepared using chiral synthons or chiral reagents, or resolved using conventional techniques, such as HPLC using a chiral column. When the compounds described herein contain olefinic double bonds or other centers of geometric asymmetry, and unless specified otherwise, it is intended that the compounds include both E and Z geometric isomers. Likewise, all tautomeric forms are also intended to be included.

In the present disclosure, a "stereoisomer" refers to a compound made up of the same atoms bonded by the same bonds but having different three-dimensional structures, which are not interchangeable. The present disclosure contemplates various stereoisomers and mixtures thereof.

### DETAILED DESCRIPTION

In one aspect, the present disclosure relates to a method for treating moderate to severe plaque psoriasis, comprising administering to a subject in need thereof a therapeutically effective amount of a compound of Formula (I), a stereoisomer thereof, or a pharmaceutically acceptable salt thereof,

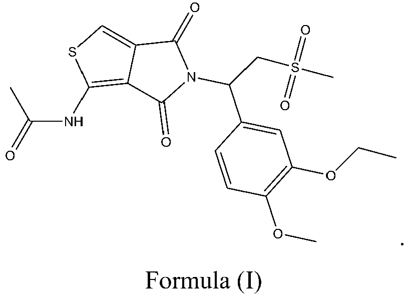

In some embodiments, the compound of Formula (I), a stereoisomer thereof, or a pharmaceutically acceptable salt thereof is administered to the subject at a daily dose of 30 mg to 180 mg.

In some embodiments, the compound of Formula (I), a stereoisomer thereof, or a pharmaceutically acceptable salt thereof is administered to the subject at a daily dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, or 180 mg.

In some embodiments, the compound of Formula (I), a stereoisomer thereof, or a pharmaceutically acceptable salt thereof is administered to the subject at a daily dose of 60 mg to 150 mg.

In some embodiments, the compound of Formula (I), a stereoisomer thereof, or a pharmaceutically acceptable salt thereof is administered to the subject at a daily dose of 60 mg to 120 mg.

In some embodiments, the compound of Formula (I), a stereoisomer thereof, or a pharmaceutically acceptable salt thereof is administered to the subject at a daily dose of 30 mg, 60 mg, 120 mg, or 150 mg.

In some embodiments, the compound of Formula (I), a stereoisomer thereof, or a pharmaceutically acceptable salt thereof is administered to the subject at a daily dose of 120 mg.

In some embodiments, the compound of Formula (I), a stereoisomer thereof, or a pharmaceutically acceptable salt thereof is administered to the subject at a daily dose of 150 mg.

In some embodiments, the compound of Formula (I), a stereoisomer thereof, or a pharmaceutically acceptable salt thereof is administered to the subject at least once a day.

In some embodiments, the compound of Formula (I), a stereoisomer thereof, or a pharmaceutically acceptable salt thereof is administered to the subject at least twice a day.

In some embodiments, the compound of Formula (I), a stereoisomer thereof, or a pharmaceutically acceptable salt thereof is administered to the subject twice a day.

In some embodiments, the compound of Formula (I), a stereoisomer thereof, or a pharmaceutically acceptable salt thereof is administered to the subject three times a day.

In some embodiments, the compound of Formula (I), a stereoisomer thereof, or a pharmaceutically acceptable salt thereof is administered to the subject twice daily at a dose of 30 mg to 75 mg per administration.

In some embodiments, the compound of Formula (I), a stereoisomer thereof, or a pharmaceutically acceptable salt thereof is administered to the subject twice daily at a dose of 30 mg to 60 mg per administration.

In some embodiments, exemplary examples of unit dose of the compound of Formula (I), a stereoisomer thereof, or a pharmaceutically acceptable salt thereof that can be used in the present disclosure include, but are not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg, 150 mg.

In some embodiments, the unit dose of the compound of Formula (I), a stereoisomer thereof, or a pharmaceutically acceptable salt thereof is 15 mg to 150 mg.

In some embodiments, the unit dose of the compound of Formula (I), a stereoisomer thereof, or a pharmaceutically acceptable salt thereof is 15 mg to 75 mg.

In some embodiments, the unit dose of the compound of Formula (I), a stereoisomer thereof, or a pharmaceutically acceptable salt thereof is 15 mg, 30 mg, 45 mg, 60 mg, 75 mg, 120 mg, and 150 mg.

In some embodiments, the compound of Formula (I), a stereoisomer thereof, or a pharmaceutically acceptable salt thereof is administered to the subject in the form of a tablet, a capsule and the like.

In some embodiments, the compound of Formula (I), a stereoisomer thereof, or a pharmaceutically acceptable salt thereof is administered orally to the subject.

In some embodiments, exemplary examples of subject that can be used in the present disclosure include, but are not limited to, a mammal.

In some embodiments, exemplary examples of mammal that can be used in the present disclosure include, but are not limited to, dog, cat, cattle, sheep, horse, and human.

In some embodiments, the subject is a human.

In some embodiments, the subject suffers from a latent tuberculosis infection.

In some embodiments, the subject has a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis.

In some embodiments, the compound of Formula (I), a stereoisomer thereof, or a pharmaceutically acceptable salt thereof of the present disclosure has good compliance in the treatment of moderate to severe plaque psoriasis.

In some embodiments, titrated dosing of the compound of Formula (I), a stereoisomer thereof, or a pharmaceutically acceptable salt thereof of the present disclosure is not required in the treatment of moderate to severe plaque psoriasis.

In some embodiments, the compound of Formula (I), a stereoisomer thereof, or a pharmaceutically acceptable salt thereof of the present disclosure is administered to a subject for short-term or long-term administration while no depression is caused in the subject.

In some embodiments, the compound of Formula (I), a stereoisomer thereof, or a pharmaceutically acceptable salt thereof of the present disclosure is administered to a subject for short-term or long-term administration while no depression is exacerbated in the subject.

In some embodiments, the compound of Formula (I), a stereoisomer thereof, or a pharmaceutically acceptable salt thereof of the present disclosure has a rapid onset of action in the treatment of moderate to severe plaque psoriasis.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent.

In some embodiments, in the treatment of moderate to severe plaque psoriasis with the compound of Formula (I), a stereoisomer thereof, or a pharmaceutically acceptable salt thereof of the present disclosure, the subject has a reduction in Psoriatic Body Surface Area, and/or a reduction in Psoriasis Area and Severity Index, and/or a reduction in Dermatology Life Quality Index.

In some embodiments, in the treatment of moderate to severe plaque psoriasis with the compound of Formula (I), a stereoisomer thereof, or a pharmaceutically acceptable salt thereof of the present disclosure, the subject's sPGA (Static Physician Global Assessment) is improved, in particular can reach a score of 0 or 1.

In some embodiments, in the treatment of moderate to severe plaque psoriasis with the compound of Formula (I), a stereoisomer thereof, or a pharmaceutically acceptable salt thereof of the present disclosure, no anxiety is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, no depressive mood is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, immunity of the subject is not reduced.

In some embodiments, the compound of Formula (I), a stereoisomer thereof, or a pharmaceutically acceptable salt thereof of the present disclosure has good safety in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good tolerability in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good compliance in the treatment of moderate to severe plaque psoriasis in a subject with latent tuberculosis infection.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has a rapid onset of action in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject with latent tuberculosis infection.

In some embodiments, the compound of Formula (I), a stereoisomer thereof, or a pharmaceutically acceptable salt thereof of the present disclosure has an improved therapeutic effect in the treatment of moderate to severe plaque psoriasis in a subject who has failed to respond to a biological agent and has latent tuberculosis infection.

In some embodiments, the compound of Formula (I), a stereoisomer thereof, or a pharmaceutically acceptable salt thereof of the present disclosure can be used in the treatment of moderate to severe plaque psoriasis in a subject with latent tuberculosis infection, either for short-term or long-term administration, causing no depression in the subject.

In some embodiments, the compound of Formula (I), a stereoisomer thereof, or a pharmaceutically acceptable salt thereof of the present disclosure can be used in the treatment of moderate to severe plaque psoriasis in a subject with latent tuberculosis infection, and latent tuberculosis infection is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, immunity of the subject is not reduced.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject has a reduction in Psoriasis Body Surface Area, and/or a reduction in Psoriasis Area and Severity Index, and/or a reduction in Dermatology Life Quality Index.

In some embodiments, the compound of Formula (I), a stereoisomer thereof, or a pharmaceutically acceptable salt thereof of the present disclosure has good safety in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good compliance in the treatment of moderate to severe plaque psoriasis in a subject with a medical history of pulmonary tuberculosis infection, tuberculosis, and/or tuberculous pleurisy.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has a rapid onset of action in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent and has a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure can be used in the treatment of moderate to severe plaque psoriasis in a subject with a medical history of pulmonary tuberculosis infection, tuberculosis, or tuberculous pleurisy, either for short-term or long-term administration, causing no depression in the subject.

In some embodiments, the compound of Formula (I), a stereoisomer thereof, or a pharmaceutically acceptable salt thereof of the present disclosure can be used in the treatment of moderate to severe plaque psoriasis in a subject with a medical history of pulmonary tuberculosis infection, tuberculosis, and/or tuberculous pleurisy, and pulmonary tuberculosis infection, tuberculosis, and/or tuberculous pleurisy is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, or tuberculous pleurisy, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, immunity of the subject is not reduced.

In another aspect, the present disclosure relates to a compound of Formula (I), a stereoisomer thereof, or a pharmaceutically acceptable salt thereof for use in treating moderate to severe plaque psoriasis in a subject,

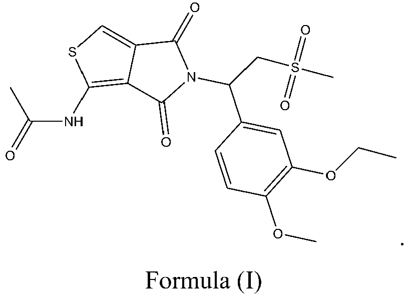

In some embodiments, the compound of Formula (I), a stereoisomer thereof, or a pharmaceutically acceptable salt thereof is administered to the subject at a daily dose of 30 mg to 180 mg.

In some embodiments, the compound of Formula (I), a stereoisomer thereof, or a pharmaceutically acceptable salt thereof is administered to the subject at a daily dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, or 180 mg.

In some embodiments, the compound of Formula (I), a stereoisomer thereof, or a pharmaceutically acceptable salt thereof is administered to the subject at a daily dose of 30 mg to 150 mg.

In some embodiments, the compound of Formula (I), a stereoisomer thereof, or a pharmaceutically acceptable salt thereof is administered to the subject at a daily dose of 60 mg to 120 mg.

In some embodiments, the compound of Formula (I), a stereoisomer thereof, or a pharmaceutically acceptable salt thereof is administered to the subject at a daily dose of 30 mg, 60 mg, 120 mg, or 150 mg.

In some embodiments, the compound of Formula (I), a stereoisomer thereof, or a pharmaceutically acceptable salt thereof is administered to the subject at a daily dose of 120 mg.

In some embodiments, the compound of Formula (I), a stereoisomer thereof, or a pharmaceutically acceptable salt thereof is administered to the subject at a daily dose of 150 mg.

In some embodiments, the compound of Formula (I), a stereoisomer thereof, or a pharmaceutically acceptable salt thereof is administered to the subject at least once a day.

In some embodiments, the compound of Formula (I), a stereoisomer thereof, or a pharmaceutically acceptable salt thereof is administered to the subject at least twice a day.

In some embodiments, the compound of Formula (I), a stereoisomer thereof, or a pharmaceutically acceptable salt thereof is administered to the subject twice a day.

In some embodiments, the compound of Formula (I), a stereoisomer thereof, or a pharmaceutically acceptable salt thereof is administered to the subject three times a day.

In some embodiments, the compound of Formula (I), a stereoisomer thereof, or a pharmaceutically acceptable salt thereof is administered to the subject twice daily at a dose of 30 mg to 75 mg per administration.

In some embodiments, the compound of Formula (I), a stereoisomer thereof, or a pharmaceutically acceptable salt thereof is administered to the subject twice daily at a dose of 30 mg to 60 mg per administration.

In some embodiments, exemplary examples of unit dose of the compound of Formula (I), a stereoisomer thereof, or a pharmaceutically acceptable salt thereof that can be used in the present disclosure include, but are not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg, 150 mg.

In some embodiments, the unit dose of the compound of Formula (I), a stereoisomer thereof, or a pharmaceutically acceptable salt thereof is 15 mg to 150 mg.

In some embodiments, the unit dose of the compound of Formula (I), a stereoisomer thereof, or a pharmaceutically acceptable salt thereof is 15 mg to 75 mg.

In some embodiments, the unit dose of the compound of Formula (I), a stereoisomer thereof, or a pharmaceutically acceptable salt thereof is 15 mg, 30 mg, 45 mg, 60 mg, 75 mg, 120 mg, and 150 mg.

In some embodiments, the compound of Formula (I), a stereoisomer thereof, or a pharmaceutically acceptable salt thereof is administered to the subject in the form of a tablet, a capsule and the like.

In some embodiments, the compound of Formula (I), a stereoisomer thereof, or a pharmaceutically acceptable salt thereof is administered orally to the subject.

In some embodiments, exemplary examples of subject that can be used in the present disclosure include, but are not limited to, a mammal.

In some embodiments, exemplary examples of mammal that can be used in the present disclosure include, but are not limited to, dog, cat, cattle, sheep, horse, and human.

In some embodiments, the subject is a human.

In some embodiments, the subject suffers from a latent tuberculosis infection.

In some embodiments, the subject has a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis.

In some embodiments, the compound of Formula (I), a stereoisomer thereof, or a pharmaceutically acceptable salt thereof of the present disclosure has good compliance in the treatment of moderate to severe plaque psoriasis.

In some embodiments, titrated dosing of the compound of Formula (I), a stereoisomer thereof, or a pharmaceutically acceptable salt thereof of the present disclosure is not required in the treatment of moderate to severe plaque psoriasis.

In some embodiments, the compound of Formula (I), a stereoisomer thereof, or a pharmaceutically acceptable salt thereof of the present disclosure is administered to a subject for short-term or long-term administration while no depression is caused in the subject.

In some embodiments, the compound of Formula (I), a stereoisomer thereof, or a pharmaceutically acceptable salt thereof of the present disclosure is administered to a subject for short-term or long-term administration while no depression is exacerbated in the subject.

In some embodiments, the compound of Formula (I), a stereoisomer thereof, or a pharmaceutically acceptable salt thereof of the present disclosure has a rapid onset of action in the treatment of moderate to severe plaque psoriasis.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent.

In some embodiments, in the treatment of moderate to severe plaque psoriasis with the compound of Formula (I), a stereoisomer thereof, or a pharmaceutically acceptable salt thereof of the present disclosure, the subject has a reduction in Psoriasis Body Surface Area, and/or a reduction in Psoriasis Area and Severity Index, and/or a reduction in Dermatology Life Quality Index.

In some embodiments, in the treatment of moderate to severe plaque psoriasis with the compound of Formula (I), a stereoisomer thereof, or a pharmaceutically acceptable salt thereof of the present disclosure, the subject's sPGA (Static Physician Global Assessment) is improved, in particular can reach a score of 0 or 1.

In some embodiments, in the treatment of moderate to severe plaque psoriasis with the compound of Formula (I), a stereoisomer thereof, or a pharmaceutically acceptable salt thereof of the present disclosure, no anxiety is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, no depressive mood is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, immunity of the subject is not reduced.

In some embodiments, the compound of Formula (I), a stereoisomer thereof, or a pharmaceutically acceptable salt thereof of the present disclosure has good safety in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good tolerability in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good compliance in the treatment of moderate to severe plaque psoriasis in a subject with latent tuberculosis infection.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has a rapid onset of action in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject with latent tuberculosis infection.

In some embodiments, the compound of Formula (I), a stereoisomer thereof, or a pharmaceutically acceptable salt thereof of the present disclosure has an improved therapeutic effect in the treatment of moderate to severe plaque psoriasis in a subject who has failed to respond to a biological agent and has latent tuberculosis infection.

In some embodiments, the compound of Formula (I), a stereoisomer thereof, or a pharmaceutically acceptable salt thereof of the present disclosure can be used in the treatment of moderate to severe plaque psoriasis in a subject with latent tuberculosis infection, and latent tuberculosis infection is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, immunity of the subject is not reduced.

In some embodiments, the compound of Formula (I), a stereoisomer thereof, or a pharmaceutically acceptable salt thereof of the present disclosure has good safety in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good compliance in the treatment of moderate to severe plaque psoriasis in a subject with a medical history of pulmonary tuberculosis infection, tuberculosis, and/or tuberculous pleurisy.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has a rapid onset of action in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent and has a medical history of pulmonary tuberculosis infection, tuberculosis, and/or tuberculous pleurisy.

In some embodiments, in the treatment of moderate to severe plaque psoriasis in a subject with a medical history of pulmonary tuberculosis infection, tuberculosis, and/or tuberculous pleurisy with the compound of Formula (I), a stereoisomer thereof, or a pharmaceutically acceptable salt thereof of the present disclosure, the pulmonary tuberculosis infection, tuberculosis, and/or tuberculous pleurisy is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, immunity of the subject is not reduced.

In a further aspect, the present disclosure relates to use of a compound of Formula (I), a stereoisomer thereof, or a pharmaceutically acceptable salt thereof in the manufacture of a medicament for treatment of moderate to severe plaque psoriasis in a subject,

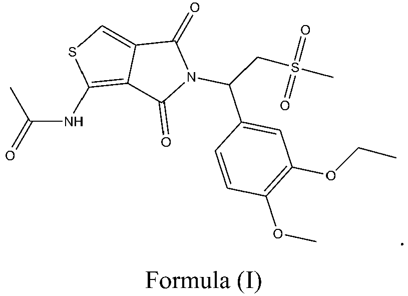

In some embodiments, the compound of Formula (I), a stereoisomer thereof, or a pharmaceutically acceptable salt thereof is administered to the subject at a daily dose of 30 mg to 180 mg.

In some embodiments, the compound of Formula (I), a stereoisomer thereof, or a pharmaceutically acceptable salt thereof is administered to the subject at a daily dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, or 180 mg.

In some embodiments, the compound of Formula (I), a stereoisomer thereof, or a pharmaceutically acceptable salt thereof is administered to the subject at a daily dose of 30 mg to 150 mg.

In some embodiments, the compound of Formula (I), a stereoisomer thereof, or a pharmaceutically acceptable salt thereof is administered to the subject at a daily dose of 60 mg to 120 mg.

In some embodiments, the compound of Formula (I), a stereoisomer thereof, or a pharmaceutically acceptable salt thereof is administered to the subject at a daily dose of 30 mg, 60 mg, 120 mg, or 150 mg.

In some embodiments, the compound of Formula (I), a stereoisomer thereof, or a pharmaceutically acceptable salt thereof is administered to the subject at a daily dose of 120 mg.

In some embodiments, the compound of Formula (I), a stereoisomer thereof, or a pharmaceutically acceptable salt thereof is administered to the subject at a daily dose of 150 mg.

In some embodiments, the compound of Formula (I), a stereoisomer thereof, or a pharmaceutically acceptable salt thereof is administered to the subject at least once a day.

In some embodiments, the compound of Formula (I), a stereoisomer thereof, or a pharmaceutically acceptable salt thereof is administered to the subject at least twice a day.

In some embodiments, the compound of Formula (I), a stereoisomer thereof, or a pharmaceutically acceptable salt thereof is administered to the subject twice a day.

In some embodiments, the compound of Formula (I), a stereoisomer thereof, or a pharmaceutically acceptable salt thereof is administered to the subject three times a day.

In some embodiments, the compound of Formula (I), a stereoisomer thereof, or a pharmaceutically acceptable salt thereof is administered to the subject twice daily at a dose of 30 mg to 75 mg per administration.

In some embodiments, the compound of Formula (I), a stereoisomer thereof, or a pharmaceutically acceptable salt thereof is administered to the subject twice daily at a dose of 30 mg to 60 mg per administration.

In some embodiments, exemplary examples of unit dose of the compound of Formula (I), a stereoisomer thereof, or a pharmaceutically acceptable salt thereof that can be used in the present disclosure include, but are not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg, 150 mg.

In some embodiments, the unit dose of the compound of Formula (I), a stereoisomer thereof, or a pharmaceutically acceptable salt thereof is 15 mg to 150 mg.

In some embodiments, the unit dose of the compound of Formula (I), a stereoisomer thereof, or a pharmaceutically acceptable salt thereof is 15 mg to 75 mg.

In some embodiments, the unit dose of the compound of Formula (I), a stereoisomer thereof, or a pharmaceutically acceptable salt thereof is 15 mg, 30 mg, 45 mg, 60 mg, 75 mg, 120 mg, and 150 mg.

In some embodiments, the compound of Formula (I), a stereoisomer thereof, or a pharmaceutically acceptable salt thereof is administered to the subject in the form of a tablet, a capsule and the like.

In some embodiments, the compound of Formula (I), a stereoisomer thereof, or a pharmaceutically acceptable salt thereof is administered orally to the subject.

In some embodiments, exemplary examples of subject that can be used in the present disclosure include, but are not limited to, a mammal.

In some embodiments, exemplary examples of mammal that can be used in the present disclosure include, but are not limited to, dog, cat, cattle, sheep, horse, and human.

In some embodiments, the subject is a human.

In some embodiments, the subject suffers from a latent tuberculosis infection.

In some embodiments, the subject has a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis.

In some embodiments, the compound of Formula (I), a stereoisomer thereof, or a pharmaceutically acceptable salt thereof of the present disclosure has good compliance in the treatment of moderate to severe plaque psoriasis.

In some embodiments, titrated dosing of the compound of Formula (I), a stereoisomer thereof, or a pharmaceutically acceptable salt thereof of the present disclosure is not required in the treatment of moderate to severe plaque psoriasis.

In some embodiments, the compound of Formula (I), a stereoisomer thereof, or a pharmaceutically acceptable salt thereof of the present disclosure is administered to a subject for short-term or long-term administration while no depression is caused in the subject.

In some embodiments, the compound of Formula (I), a stereoisomer thereof, or a pharmaceutically acceptable salt thereof of the present disclosure is administered to a subject for short-term or long-term administration while no depression is exacerbated in the subject.

In some embodiments, the compound of Formula (I), a stereoisomer thereof, or a pharmaceutically acceptable salt thereof of the present disclosure has a rapid onset of action in the treatment of moderate to severe plaque psoriasis.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent.

In some embodiments, in the treatment of moderate to severe plaque psoriasis with the compound of Formula (I), a stereoisomer thereof, or a pharmaceutically acceptable salt thereof of the present disclosure, the subject has a reduction in Psoriasis Body Surface Area, and/or a reduction in Psoriasis Area and Severity Index, and/or a reduction in Dermatology Life Quality Index.

In some embodiments, in the treatment of moderate to severe plaque psoriasis with the compound of Formula (I), a stereoisomer thereof, or a pharmaceutically acceptable salt thereof of the present disclosure, the subject's sPGA (Static Physician Global Assessment) is improved, in particular can reach a score of 0 or 1.

In some embodiments, in the treatment of moderate to severe plaque psoriasis with the compound of Formula (I), a stereoisomer thereof, or a pharmaceutically acceptable salt thereof of the present disclosure, no anxiety is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, no depressive mood is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, immunity of the subject is not reduced.

In some embodiments, the compound of Formula (I), a stereoisomer thereof, or a pharmaceutically acceptable salt thereof of the present disclosure has good safety in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good tolerability in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good compliance in the treatment of moderate to severe plaque psoriasis in a subject with latent tuberculosis infection.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has a rapid onset of action in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject with latent tuberculosis infection.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject with latent tuberculosis infection.

In some embodiments, the compound of Formula (I), a stereoisomer thereof, or a pharmaceutically acceptable salt thereof of the present disclosure has an improved therapeutic effect in the treatment of moderate to severe plaque psoriasis in a subject who has failed to respond to a biological agent and has latent tuberculosis infection.

In some embodiments, the compound of Formula (I), a stereoisomer thereof, or a pharmaceutically acceptable salt thereof of the present disclosure can be used in the treatment of moderate to severe plaque psoriasis in a subject with latent tuberculosis infection, and latent tuberculosis infection is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, immunity of the subject is not reduced.

In some embodiments, the compound of Formula (I), a stereoisomer thereof, or a pharmaceutically acceptable salt thereof of the present disclosure has good safety in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good compliance in the treatment of moderate to severe plaque psoriasis in a subject with a medical history of pulmonary tuberculosis infection, tuberculosis, and/or tuberculous pleurisy.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has a rapid onset of action in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent and has a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure can be used in the treatment of moderate to severe plaque psoriasis in a subject with a medical history of pulmonary tuberculosis infection, tuberculosis, and/or tuberculous pleurisy, and pulmonary tuberculosis infection, tuberculosis, and/or tuberculous pleurisy is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, immunity of the subject is not reduced.

In yet another aspect, the present disclosure relates to a pharmaceutical composition for treating moderate to severe plaque psoriasis in a subject, comprising a compound of Formula (I), a stereoisomer thereof, or a pharmaceutically acceptable salt thereof, and a pharmaceutically acceptable carrier, diluent, or excipient,

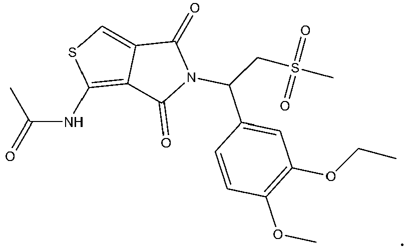

In some embodiments, the compound of Formula (I), a stereoisomer thereof, or a pharmaceutically acceptable salt thereof is administered to the subject at a daily dose of 30 mg to 180 mg.

In some embodiments, the compound of Formula (I), a stereoisomer thereof, or a pharmaceutically acceptable salt thereof is administered to the subject at a daily dose of 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, or 180 mg.

In some embodiments, the compound of Formula (I), a stereoisomer thereof, or a pharmaceutically acceptable salt thereof is administered to the subject at a daily dose of 60 mg to 150 mg.

In some embodiments, the compound of Formula (I), a stereoisomer thereof, or a pharmaceutically acceptable salt thereof is administered to the subject at a daily dose of 60 mg to 120 mg.

In some embodiments, the compound of Formula (I), a stereoisomer thereof, or a pharmaceutically acceptable salt thereof is administered to the subject at a daily dose of 30 mg, 60 mg, 120 mg, or 150 mg.

In some embodiments, the compound of Formula (I), a stereoisomer thereof, or a pharmaceutically acceptable salt thereof is administered to the subject at a daily dose of 120 mg.

In some embodiments, the compound of Formula (I), a stereoisomer thereof, or a pharmaceutically acceptable salt thereof is administered to the subject at a daily dose of 150 mg.

In some embodiments, the compound of Formula (I), a stereoisomer thereof, or a pharmaceutically acceptable salt thereof is administered to the subject at least once a day.

In some embodiments, the compound of Formula (I), a stereoisomer thereof, or a pharmaceutically acceptable salt thereof is administered to the subject at least twice a day.

In some embodiments, the compound of Formula (I), a stereoisomer thereof, or a pharmaceutically acceptable salt thereof is administered to the subject twice a day.

In some embodiments, the compound of Formula (I), a stereoisomer thereof, or a pharmaceutically acceptable salt thereof is administered to the subject three times a day.

In some embodiments, the compound of Formula (I), a stereoisomer thereof, or a pharmaceutically acceptable salt thereof is administered to the subject twice daily at a dose of 30 mg to 75 mg per administration.

In some embodiments, the compound of Formula (I), a stereoisomer thereof, or a pharmaceutically acceptable salt thereof is administered to the subject twice daily at a dose of 30 mg to 60 mg per administration.

In some embodiments, exemplary examples of unit dose of the compound of Formula (I), a stereoisomer thereof, or a pharmaceutically acceptable salt thereof that can be used in the present disclosure include, but are not limited to, 2.5 mg, 5 mg, 7.5 mg, 10 mg, 15 mg, 20 mg, 30 mg, 40 mg, 45 mg, 50 mg, 60 mg, 70 mg, 75 mg, 80 mg, 90 mg, 100 mg, 110 mg, 120 mg, 130 mg, 140 mg, 150 mg.

In some embodiments, the unit dose of the compound of Formula (I), a stereoisomer thereof, or a pharmaceutically acceptable salt thereof is 15 mg to 150 mg.

In some embodiments, the unit dose of the compound of Formula (I), a stereoisomer thereof, or a pharmaceutically acceptable salt thereof is 15 mg to 75 mg.

In some embodiments, the unit dose of the compound of Formula (I), a stereoisomer thereof, or a pharmaceutically acceptable salt thereof is 15 mg, 30 mg, 45 mg, 60 mg, 75 mg, 120 mg, and 150 mg.

In some embodiments, the compound of Formula (I), a stereoisomer thereof, or a pharmaceutically acceptable salt thereof is administered to the subject in the form of a tablet, a capsule and the like.

In some embodiments, the compound of Formula (I), a stereoisomer thereof, or a pharmaceutically acceptable salt thereof is administered orally to the subject.

In some embodiments, exemplary examples of subject that can be used in the present disclosure include, but are not limited to, a mammal.

In some embodiments, exemplary examples of mammal that can be used in the present disclosure include, but are not limited to, dog, cat, cattle, sheep, horse, and human.

In some embodiments, the subject is a human.

In some embodiments, the subject suffers from a latent tuberculosis infection.

In some embodiments, the subject has a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis.

In some embodiments, the compound of Formula (I), a stereoisomer thereof, or a pharmaceutically acceptable salt thereof of the present disclosure has good compliance in the treatment of moderate to severe plaque psoriasis.

In some embodiments, titrated dosing of the compound of Formula (I), a stereoisomer thereof, or a pharmaceutically acceptable salt thereof of the present disclosure is not required in the treatment of moderate to severe plaque psoriasis.

In some embodiments, the compound of Formula (I), a stereoisomer thereof, or a pharmaceutically acceptable salt thereof of the present disclosure is administered to a subject for short-term or long-term administration while no depression is caused in the subject.

In some embodiments, the compound of Formula (I), a stereoisomer thereof, or a pharmaceutically acceptable salt thereof of the present disclosure is administered to a subject for short-term or long-term administration while no depression is exacerbated in the subject.

In some embodiments, the compound of Formula (I), a stereoisomer thereof, or a pharmaceutically acceptable salt thereof of the present disclosure has a rapid onset of action in the treatment of moderate to severe plaque psoriasis.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent.

In some embodiments, in the treatment of moderate to severe plaque psoriasis with the compound of Formula (I), a stereoisomer thereof, or a pharmaceutically acceptable salt thereof of the present disclosure, the subject has a reduction in Psoriasis Body Surface Area, and/or a reduction in Psoriasis Area and Severity Index, and/or a reduction in Dermatology Life Quality Index.

In some embodiments, in the treatment of moderate to severe plaque psoriasis with the compound of Formula (I), a stereoisomer thereof, or a pharmaceutically acceptable salt thereof of the present disclosure, the subject's sPGA (Static Physician Global Assessment) is improved, in particular can reach a score of 0 or 1.

In some embodiments, in the treatment of moderate to severe plaque psoriasis with the compound of Formula (I), a stereoisomer thereof, or a pharmaceutically acceptable salt thereof of the present disclosure, no anxiety is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, no depressive mood is caused or exacerbated in the subject.

In some embodiments, in the treatment of moderate to severe plaque psoriasis with the compound of Formula (I), a stereoisomer thereof, or a pharmaceutically acceptable salt thereof of the present disclosure, immunity of the subject is not reduced.

In some embodiments, the compound of Formula (I), a stereoisomer thereof, or a pharmaceutically acceptable salt thereof of the present disclosure has good safety in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good tolerability in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has good compliance in the treatment of moderate to severe plaque psoriasis in a subject with latent tuberculosis infection.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure has a rapid onset of action in the treatment of moderate to severe plaque psoriasis in a subject with latent tuberculosis infection.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject.

In some embodiments, the compound of Formula (I), a stereoisomer thereof, or a pharmaceutically acceptable salt thereof of the present disclosure has an improved therapeutic effect in the treatment of moderate to severe plaque psoriasis in a subject who has failed to respond to a biological agent and has latent tuberculosis infection.

In some embodiments, the compound of Formula (I), a stereoisomer thereof, or a pharmaceutically acceptable salt thereof of the present disclosure is administered to a subject with latent tuberculosis infection, either for short-term or long-term administration, causing no depression in the subject.

In some embodiments, the compound of Formula (I), a stereoisomer thereof, or a pharmaceutically acceptable salt thereof of the present disclosure can be used in the treatment of moderate to severe plaque psoriasis in a subject with latent tuberculosis infection, and latent tuberculosis infection is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with latent tuberculosis infection, immunity of the subject is not reduced.

In some embodiments, the compound of Formula (I), a stereoisomer thereof, or a pharmaceutically acceptable salt thereof of the present disclosure has good safety in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good tolerability in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure has good compliance in the treatment of moderate to severe plaque psoriasis in a subject with a medical history of pulmonary tuberculosis infection, tuberculosis, and/or tuberculous pleurisy.

In some embodiments, titrated dosing of the compound of Formula (I), a stereoisomer thereof, or a pharmaceutically acceptable salt thereof of the present disclosure is not required in the treatment of moderate to severe plaque psoriasis in a subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-naïve subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a biological agent-experienced subject with a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure also has an improved therapeutic effect in the treatment of moderate to severe plaque psoriasis in a subject who has failed to respond to a biological agent and has a medical history of pulmonary tuberculosis infection, tuberculosis, and/or tuberculous pleurisy.

In some embodiments, the compound of Formula (I), a stereoisomer thereof, or a pharmaceutically acceptable salt thereof of the present disclosure can be used in the treatment of moderate to severe plaque psoriasis in a subject with a medical history of pulmonary tuberculosis infection, tuberculosis, and/or tuberculous pleurisy, and/or tuberculous pleurisy is not reactivated after treatment.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject has a reduction in Psoriasis Body Surface Area, and/or a reduction in Psoriasis Area and Severity Index, and/or a reduction in Dermatology Life Quality Index.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, the subject's sPGA (Static Physician Global Assessment) is improved, in particular can reach a score of 0 or 1.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, no anxiety is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, no depressive mood is caused or exacerbated in the subject.

In some embodiments, when the compound of Formula (I), a stereoisomer thereof, or a pharmaceutically acceptable salt thereof of the present disclosure is used in the treatment of moderate to severe plaque psoriasis in a subject with a medical history of pulmonary tuberculosis infection, tuberculosis, and/or tuberculous pleurisy, immunity of the subject is not reduced.

Hereinafter, the present disclosure is explained in detail through the following examples in order to better understand various aspects and advantages of the present disclosure. However, it should be understood that the following examples are non-limiting and are only used to illustrate some embodiments of the present disclosure.

### EXAMPLES

### Definition of Terms:

PASI: Psoriasis Area and Severity Index.
PASI Score Change Rate (PASI Improvement): Calculated as: (Baseline PASI Score - Visit PASI Score)/Baseline PASI Score ×100%.
PASI 75: ≥75% improvement from baseline in the PASI score.
PASI-90: ≥90% improvement from baseline in the PASI score.
PASI-50: ≥50% improvement from baseline in the PASI score.
sPGA: Static Physician Global Assessment; used to determine the overall severity of a patient's psoriasis at a specific time point. The assessment reflects the overall severity of induration, erythema and scaling, using specific numerical values to represent the severity of the lesions.
BSA: Psoriasis Body Surface Area (Total area of psoriasis lesions).
DLQI: Dermatology Life Quality Index.
BID: Twice daily (bis in die).
HADS: Hospital Anxiety and Depression Scale.

### Example 1

### Preparation of compound of Formula (I)

### N-[5-[1-(3-ethoxy-4-methoxyphenyl)-2-(methylsulfonyl)ethyl]-4,6-dioxo-5,6-dihydro-5H-thieno[3,4-c]pyrrol-1-yl]acetamide

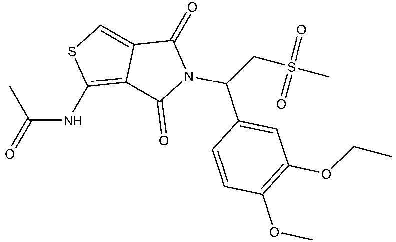

The title compound was prepared according to preparation process 2 of Example 3 in CN 101885731 A.

### Example 2

### Preparation of stereoisomer of compound of Formula (I)

### (S)-N-[5-[1-(3-ethoxy-4-methoxyphenyl)-2-(methylsulfonyl)ethyl]-4,6-dioxo-5,6-dihydro-5H-thieno[3,4-c]pyrrol-1-yl]acetamide

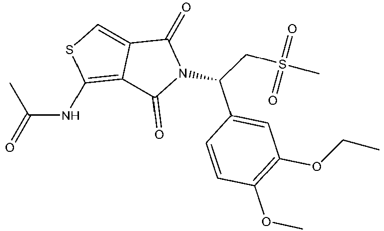

The title compound was prepared according to preparation process of Example 1 in CN 116332954 A.

### Example 3

### Clinical study on patients

This study selected patients with moderate to severe chronic plaque psoriasis as target subjects. Specific criteria were set for disease duration, severity and extent (psoriasis history ≥ 6 months, PASI score ≥ 12, sPGA score ≥ 3, and BSA ≥ 10). A total of 305 subjects (including 211 in the experimental group and 94 in the placebo group; of which 105 subjects were T-SPOT positive but without active tuberculosis) were enrolled. Primary efficacy endpoint: PASI-75 at week 16. Secondary efficacy endpoints: An sPGA score of 0 or 1 with a reduction of ≥ 2 points from baseline; PASI-90; PASI-50; sPGA score; both PASI-75 and sPGA 0/1 with a ≥ 2-point reduction from baseline; and recurrence rate. Improvement in pruritus symptoms and changes in DLQI scores were also evaluated. The study consisted of a 16-week core treatment period, a 36-week extension treatment period and a 4-week follow-up period. Subjects in the experimental group received 60 mg BID of the compound in Example 2. During the extension treatment period, subjects in the placebo group also received 60 mg BID of the compound in Example 2.

### Results of this clinical study:

### 1. Efficacy

Primary Efficacy Indicator: At week 16, the proportion of subjects achieving PASI-75 was 53.6% in the Example 2 group, compared to 16.0% in the placebo group. The difference was statistically significant (P<0.0001), with a rate difference of 37.6% between the two groups, demonstrating that the efficacy of Example 2 is superior to placebo.

Secondary Efficacy Indicators: The Example 2 group also showed statistically significant differences compared to the placebo group (P<0.0001), further supporting the efficacy of the compound in Example 2.

### 2. Safety

The severity of drug-related adverse events was primarily Grade 1 or Grade 2.

No deaths occurred.

No drug-related serious adverse events occurred.

Adverse events leading to discontinuation were all Grade 1 or Grade 2 in severity. No adverse events of Grade 3 or higher led to discontinuation.

By the end of the clinical study, no tuberculosis reactivation occurred among the T-SPOT positive but non-active TB subjects. Chest imaging examinations at the week 16 visit showed no abnormalities.

By the end of the clinical study, no occurrences of depression or suicidal ideation were observed in any subjects.

**Table 2. Clinical response information of 16-week core treatment period:**

| Evaluation Indicator | | Example 2 60 mg BID | Placebo | Statistical Difference |
|---|---|---|---|---|
| | | N=211 | N=94 | |
| Main Indicator | PASI-75 | 53.6% | 16.0% | P<0.0001 |
| Secondary Indicator | PASI-90 | 32.5% | 5.8% | P<0.0001 |
| | DLQI (LS mean) | 5.38 points | 2.52 points | P<0.0001 |

### Example 4

### 1. Investigational Drug

Tablets of the compound shown in Example 2 with a strength of 15 mg per tablet.

Control Drug: Placebo tablets matching the investigational drug with a strength of 0 mg per tablet.

### 2. Inclusion Criteria

1) Aged ≥ 18 years at the time of signing the Informed Consent Form (ICF), of any gender.
2) Clinically diagnosed with stable plaque psoriasis, with a psoriasis history of ≥ 6 months (prior to randomization).
3) The following requirements are met at screening and baseline:
   a. Psoriasis Area and Severity Index (PASI) score ≥ 12; and
   b. Static Physician Global Assessment (sPGA) score ≥ 3 (based on an sPGA 0-4 scale); and
   c. Psoriasis Body Surface Area (BSA) ≥ 10%.
4) Female subjects of childbearing potential and male subjects who have not undergone a vasectomy must be willing to use effective contraception throughout the study period, starting from the signing of the ICF until 3 months after the last dose of the study drug. Effective contraception includes: vasectomy, abstinence, intrauterine device (IUD), hormonal methods (oral, patch, ring, injection, implant), and barrier methods (diaphragm, cervical cap, sponge, condom). Female subjects of childbearing potential must have a negative serum pregnancy test (Human Chorionic Gonadotropin [HCG]) within 7 days prior to randomization. Male subjects must not donate sperm from the first dose until 3 months after the last dose of the study drug.

Note: Childbearing potential is defined as: a non-menopausal female who has experienced menarche and has not undergone sterilization (hysterectomy, bilateral oophorectomy, or bilateral tubal ligation), or a non-menopausal female who has undergone sterilization for less than 6 months (menopause is defined as spontaneous amenorrhea for ≥12 consecutive months).

5) Subjects participated voluntarily and signed the Informed Consent Form.

### 3. Dosing Regimen

The treatment period consisted of two phases: during the double-blind core treatment period, subjects received 60 mg BID of the investigational drug or placebo for 16 weeks (week 0 to week 16); during the open-label extension treatment period, all subjects received 60 mg BID of the investigational drug for 36 weeks (week 16 to week 52).

### 4. Study Results

A total of 305 subjects were enrolled (211 in the experimental group and 94 in the placebo group; including 105 T-SPOT positive subjects without active tuberculosis, 7 subjects with previously confirmed pulmonary tuberculosis, 76 subjects with a history of tuberculosis, and 1 subject with a history of tuberculous pleurisy).

The primary efficacy endpoint was the proportion of subjects achieving PASI 75 at week 16 compared to baseline. The key secondary efficacy endpoint was an sPGA 0/1 with a reduction of ≥2 points from baseline at week 16. Other secondary efficacy endpoints included the proportion of subjects achieving PASI-90, PASI-75, PASI-50, and both PASI-75 and sPGA 0/1 with a ≥ 2-point reduction from baseline at each visit, as well as the rate of change in BSA. Clinical observation and evaluation were conducted over the 16-week core treatment period, the 36-week extension treatment period and the 4-week follow-up period. The Hospital Anxiety and Depression Scale (HADS) was used as an assessment tool to observe and evaluate the anxiety and depression status of subjects at screening, the end of the 16-week core period and 4 weeks after the last dose.

### 1) Efficacy

**Table 3. Clinical response at week 16 in a phase III study of moderate to severe plaque psoriasis**

| Evaluation Indicator | | Investigational Drug 60 mg BID | Placebo | Statistical Difference |
|---|---|---|---|---|
| | | N=211 | N=94 | |
| Main Indicator | PASI-75 (%) | 53.6% | 16.0% | P<0.0001 |
| | sPGA score of 0/1 and reduction of ≥2 points from baseline (%) | 31.3% | 6.4% | P<0.0001 |
| | PASI-90 (%) | 32.5% | 5.8% | P<0.0001 |
| Secondary Indicator | PASI-50 (%) | 82.7% | 37.2% | P<0.0001 |
| | DLQI change from baseline, (mean) | 5.38 points | 2.52 points | P<0.0001 |
| | Both PASI-75 and sPGA 0/1 and reduction of ≥2 points from baseline (%) | 30.3% | 6.4% | P<0.0001 |
| | BSA rate of change (%) | 65.0% | 25.6% | P=0.0087 |

### 2) Safety

The primary adverse reactions and their incidences were nausea (23.7%), diarrhea (11.4%), vomiting (10.0%), increased stool frequency (10.4%), dizziness (9.0%), headache (8.1%), hypertriglyceridemia (5.7%) and fatigue (8.5%), all of which were Grade 1 or Grade 2. No specific safety warnings or risks were identified during the clinical use of the investigational drug. Gastrointestinal reactions (such as nausea, vomiting, and diarrhea) and various nervous system abnormalities (such as dizziness and headache) mostly occurred within the first two weeks of administration. Most subjects recovered spontaneously thereafter without the need for pharmacological intervention.

No death events occurred.

No drug-related serious adverse events occurred during the core treatment period.

Adverse events leading to discontinuation: All adverse events resulting in treatment discontinuation were Grade 1 or Grade 2 in severity. No discontinuation due to Grade 3 or higher adverse events occurred.

By the end of the clinical study, no instances of tuberculosis activation (reactivation) were observed among the 105 T-SPOT positive subjects without active tuberculosis, 7 subjects with previously confirmed pulmonary tuberculosis, 76 subjects with a history of tuberculosis and 1 subject with a history of tuberculous pleurisy. No clinical abnormalities related to tuberculosis were found in chest X-ray or CT examinations for any of the subjects.

**Table 4. HADS scores in moderate to severe plaque psoriasis phase III study**

| | | Week 16 | | Week 56 | |
|---|---|---|---|---|---|
| | | Experimental group | Placebo | Experimental group | Placebo |
| Indicator | | (N=211) | (N=94) | (N=211) | (N=94) |
| HADS Anxiety Score | Mean | 2.3 | 3.0 | 3.1 | 2.4 |
| HADS Depression Score | Mean | 3.0 | 3.7 | 3.8 | 3.3 |

By the end of the clinical study, there were no statistically significant differences (P>0.05) between the experimental group and the placebo group in the mean HADS anxiety scores and mean HADS depression scores (±SD). No adverse events of depression or anxiety related to the investigational drug were observed, and there were no occurrences of depression or suicidal ideation.

By the end of the clinical study, favorable efficacy was observed in patients who had previously failed biologic therapy. Specifically, two subjects who had failed prior adalimumab treatment both achieved the PASI-50 efficacy endpoint in this study.

### Example 5

### 1. Investigational Drug

Tablets of the compound shown in Example 2 with a strength of 15 mg per tablet.

Control Drug: Placebo tablets matching the investigational drug with a strength of 0 mg per tablet.

### 2. Inclusion Criteria

1) Aged ≥18 and ≤75 years, of any gender.
2) Patients with plaque psoriasis with a psoriasis history of ≥6 months.
3) At screening and baseline, the patient must meet the following requirements: Psoriasis Area and Severity Index (PASI) score ≥12, static Physician Global Assessment (sPGA) score ≥3 (moderate to severe) and Psoriasis Body Surface Area (BSA) ≥10%.
4) Assessed by the investigator as suitable for systemic psoriasis therapy.
5) Throughout the entire study period starting from the signing of the Informed Consent Form (ICF) and within 3 months after the last dose, female subjects of childbearing potential and male subjects who have not undergone a vasectomy must use effective contraception. Effective contraception includes: vasectomy, abstinence, intrauterine device (IUD), hormonal methods (oral, patch, ring, injection, implant), and barrier methods (diaphragm, cervical cap, sponge, condom).
6) Subjects understood the informed consent of this study and voluntarily signed a written Informed Consent Form.

### 3. Dosing Regimen

This was a multicenter, randomized, double-blind, placebo-parallel controlled study. A total of 216 subjects were enrolled and randomized in a 1:1:1:1 ratio into the low-dose group (15 mg BID), mid-dose group (30 mg BID), high-dose group (60 mg BID) of the investigational drug, and the placebo group for a 16-week core treatment period. Upon completion of the core treatment, subjects in the low, mid and high-dose groups maintained their original doses for a 36-week extension treatment period. Subjects in the placebo group were switched to the mid-dose group (30 mg BID) for the 36-week extension treatment after completing the core period. All subjects completed a total of 52 weeks of treatment. Subjects who completed 52 weeks of treatment or discontinued prematurely should enter a 4-week follow-up period.

### 4. Study Results

### Primary efficacy endpoint:

Proportion of subjects achieving a ≥ 75% reduction from baseline in PASI score (PASI-75) at week 16.

### Secondary efficacy endpoints:

An sPGA 0/1 with a reduction of ≥2 points from baseline at week 16; the proportion of subjects achieving PASI-90, PASI-75, rate of change in BSA; change in PASI score, PASI-50, changes in Dermatology Life Quality Index (DLQI), and both PASI-75 and sPGA 0/1 with a ≥2-point reduction from baseline at each visit. Clinical observation and evaluation were conducted over the 16-week core treatment period, the 36-week extension treatment period and the 4-week follow-up period.

### 1. Efficacy

| | | 15mg BID Group (N=54) | 30mg BID Group (N=54) | 60mg BID Group (N=54) | Placebo Group (N=54) | Statistical Difference Between 60 mg BID and Placebo |
|---|---|---|---|---|---|---|
| Main Indicator | PASI-75 response rate (%) | 18.52 | 25.93 | 46.30 | 11.11 | <0.0001 |
| Secondary Indicator | sPGA 0/1 score (%) | 14.81 | 14.81 | 24.07 | 9.26 | 0.0331 |
| | PASI-50 response rate (%) | 42.59 | 51.85 | 62.96 | 24.07 | <0.000 |
| | PASI-90 response rate (%) | 16.67 | 11.11 | 20.37 | 5.56 | 0.0125 |
| | DLQI score change from baseline, Mean | 4.9 | 6.1 | 7.1 | 2.9 | 0.0023 |
| | PASI-75 & sPGA 0/1 score (%) | 12.96 | 14.81 | 24.07 | 7.41 | 0.0144 |

### 2. Safety

During the double-blind core treatment period (weeks 1 to 16), drug-related adverse events with an incidence of ≥5% in any of the low, medium or high-dose groups included: increased triglycerides, increased lipids, diarrhea, nausea, abdominal discomfort, hyperlipidemia, headache, and fatigue. Adverse events with an incidence of ≥10% included: diarrhea, nausea, headache, and fatigue.

No death events occurred.

No drug-related serious adverse events occurred during the core treatment period.

Adverse events leading to discontinuation: All adverse events resulting in treatment discontinuation were Grade 1 or Grade 2 in severity. No discontinuation due to Grade 3 or higher adverse events occurred.

By the end of the clinical study, no adverse events of depression or anxiety were reported in either the experimental or placebo groups, and no occurrences of depression or suicidal ideation were observed.

By the end of the clinical study, favorable efficacy was observed in patients who had failed prior biologic therapy. One subject who had failed adalimumab therapy, one subject who had failed secukinumab therapy, and one subject who had failed TNF-α monoclonal antibody (mAb) therapy all achieved the PASI-50 efficacy endpoint. One subject who had failed adalimumab therapy also achieved the PASI-75 and PASI-90 efficacy endpoints.

### 3. Other Results

Population pharmacokinetics and exposure-response analysis showed that the efficacy response rate increased with increasing doses.

### Example 6

### Clinical study on subjects

A total of 36 healthy Chinese subjects participated in a phase I clinical study, in which multiple oral doses of 15 mg BID, 30 mg BID, 60 mg BID, 75 mg BID of the compound in Example 2, or a placebo were administered, respectively. The clinical trials showed good safety and tolerability.

In the present disclosure, relational terms such as "first" and "second" are used only to distinguish one entity or operation from another entity or operation, and do not necessarily require or imply any actual relationship or order.

It can be understood from the above description that although specific embodiments of the present disclosure have been described for illustrative purposes, various modifications or improvements can be made by one skilled in the art without departing from the spirit and scope of the present disclosure. Such variations or modifications should fall within the scope of the claims appended to this disclosure.

## Claims

1. A method for treating moderate to severe plaque psoriasis, comprising administering to a subject in need thereof a therapeutically effective amount of a compound of Formula (I), a stereoisomer thereof, or a pharmaceutically acceptable salt thereof,

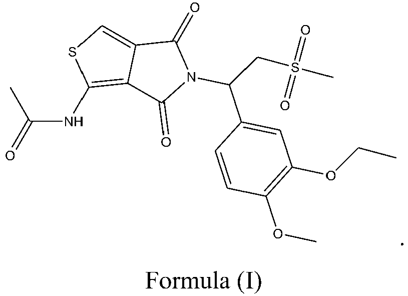

2. The method of claim 1, wherein the compound of Formula (I), a stereoisomer thereof, or a pharmaceutically acceptable salt thereof is administered to the subject at a daily dose of 30 mg to 180 mg.

3. The method of claim 1 or 2, wherein the compound of Formula (I), a stereoisomer thereof, or a pharmaceutically acceptable salt thereof is administered to the subject at a daily dose of 60 mg to 150 mg.

4. The method of claim 1 or 3, wherein the compound of Formula (I), a stereoisomer thereof, or a pharmaceutically acceptable salt thereof is administered to the subject at a daily dose of 60 mg to 120 mg.

5. The method of any one of claims 1 to 4, wherein the compound of Formula (I), a stereoisomer thereof, or a pharmaceutically acceptable salt thereof is administered to the subject at a daily dose of 120 mg.

6. The method of any one of claims 1 to 5, wherein the compound of Formula (I), a stereoisomer thereof, or a pharmaceutically acceptable salt thereof is administered to the subject at a daily dose of 150 mg.

7. The method of any one of claims 1 to 6, wherein the compound of Formula (I), a stereoisomer thereof, or a pharmaceutically acceptable salt thereof is administered to the subject at least once a day.

8. The method of any one of claims 1 to 7, wherein the compound of Formula (I), a stereoisomer thereof, or a pharmaceutically acceptable salt thereof is administered to the subject at least twice a day.

9. The method of any one of claims 1 to 8, wherein the compound of Formula (I), a stereoisomer thereof, or a pharmaceutically acceptable salt thereof is administered to the subject twice daily at a dose of 30 mg to 75 mg per administration.

10. The method of any one of claims 1 to 9, wherein the compound of Formula (I), a stereoisomer thereof, or a pharmaceutically acceptable salt thereof is administered to the subject twice daily at a dose of 30 mg to 60 mg per administration.

11. The method of any one of claims 1 to 10, wherein the unit dose of the compound of Formula (I), a stereoisomer thereof, or a pharmaceutically acceptable salt thereof is 2.5 mg to 150 mg.

12. The method of any one of claims 1 to 11, wherein the unit dose of the compound of Formula (I), a stereoisomer thereof, or a pharmaceutically acceptable salt thereof is 15 mg to 150 mg.

13. The method of any one of claims 1 to 12, wherein the unit dose of the compound of Formula (I), a stereoisomer thereof, or a pharmaceutically acceptable salt thereof is 15 mg, 30 mg, 45 mg, 60 mg, 75 mg, 120 mg, or 150 mg.

14. The method of any one of claims 1 to 13, wherein the compound of Formula (I), a stereoisomer thereof, or a pharmaceutically acceptable salt thereof is administered to the subject in the form of a tablet or a capsule.

15. The method of any one of claims 1 to 14, wherein the compound of Formula (I), a stereoisomer thereof, or a pharmaceutically acceptable salt thereof is administered orally to the subject.

16. The method of any one of claims 1 to 15, wherein the subject is a mammal, preferably a human.

17. The method of any one of claims 1 to 16, wherein the subject suffers from a latent tuberculosis infection.

18. The method of any one of claims 1 to 17, wherein the subject has a medical history of pulmonary tuberculosis infection, tuberculosis, and/or tuberculous pleurisy.

19. The method of any one of claims 1 to 18, wherein titrated dosing is not required.

20. The method of any one of claims 1 to 18, wherein the subject suffers from a latent tuberculosis infection, and latent tuberculosis infection is not reactivated after treatment.

21. The method of any one of claims 1 to 18, wherein the subject has a medical history of pulmonary tuberculosis infection, tuberculosis, and/or tuberculous pleurisy ,and pulmonary tuberculosis infection, tuberculosis, and/or tuberculous pleurisy is not reactivated after treatment.

22. The method of any one of claims 1 to 18, wherein the subject has a reduction in Psoriasis Body Surface Area, and/or a reduction in Psoriasis Area and Severity Index, and/or a reduction in Dermatology Life Quality Index.

23. The method of any one of claims 1 to 18, wherein the subject's sPGA (Static Physician Global Assessment) is improved, in particular can reach a score of 0 or 1.

24. The method of any one of claims 1 to 18, wherein no anxiety is caused or exacerbated in the subject.

25. The method of any one of claims 1 to 18, wherein no depressive mood is caused or exacerbated in the subject.

26. The method of any one of claims 1 to 18, wherein the subject also has an improved therapeutic effect in a biological agent-naive subject.

27. The method of any one of claims 1 to 18, wherein the subject also has an improved therapeutic effect in a biological agent-experienced subject.

28. The method of any one of claims 1 to 18, wherein the subject also has an improved therapeutic effect in a subject who has failed to respond to a biological agent.

29. The method of any one of claims 1 to 18, wherein immunity of the subject is not reduced.

30. A compound of Formula (I), a stereoisomer thereof, or a pharmaceutically acceptable salt thereof for use in treating moderate to severe plaque psoriasis in a subject,

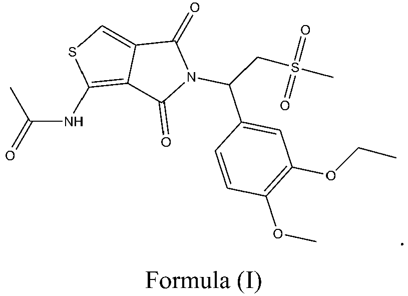

31. The compound of Formula (I), a stereoisomer thereof, or a pharmaceutically acceptable salt thereof of claim 30, wherein the compound of Formula (I), a stereoisomer thereof, or a pharmaceutically acceptable salt thereof is administered to the subject in a daily dose of 30 mg to 180 mg.

32. The compound of Formula (I), a stereoisomer thereof, or a pharmaceutically acceptable salt thereof of claim 30 or 31, wherein the compound of Formula (I), a stereoisomer thereof, or a pharmaceutically acceptable salt thereof is administered to the subject at a daily dose of 60 mg to 150 mg.

33. The compound of Formula (I), a stereoisomer thereof, or a pharmaceutically acceptable salt thereof of any one of claims 30 or 32, wherein the compound of Formula (I), a stereoisomer thereof, or a pharmaceutically acceptable salt thereof is administered to the subject at a daily dose of 60 mg to 120 mg.

34. The compound of Formula (I), a stereoisomer thereof, or a pharmaceutically acceptable salt thereof of any one of claims 30 to 33, wherein the compound of Formula (I), a stereoisomer thereof, or a pharmaceutically acceptable salt thereof is administered to the subject at a daily dose of 120 mg.

35. The compound of Formula (I), a stereoisomer thereof, or a pharmaceutically acceptable salt thereof of any one of claims 30 to 34, wherein the compound of Formula (I), a stereoisomer thereof, or a pharmaceutically acceptable salt thereof is administered to the subject at a daily dose of 150 mg.

36. The compound of Formula (I) or a pharmaceutically acceptable salt thereof of any one of claims 30 to 35, wherein the compound of Formula (I), a stereoisomer thereof, or a pharmaceutically acceptable salt thereof is administered to the subject at least once a day.

37. The compound of Formula (I) or a pharmaceutically acceptable salt thereof of any one of claims 30 to 36, wherein the compound of Formula (I), a stereoisomer thereof, or a pharmaceutically acceptable salt thereof is administered to the subject at least twice a day.

38. The compound of Formula (I) or a pharmaceutically acceptable salt thereof of any one of claims 30 to 37, wherein the compound of Formula (I), a stereoisomer thereof, or a pharmaceutically acceptable salt thereof is administered to the subject twice daily at a dose of 30 mg to 75 mg per administration.

39. The compound of Formula (I) or a pharmaceutically acceptable salt thereof of any one of claims 30 to 38, wherein the compound of Formula (I), a stereoisomer thereof, or a pharmaceutically acceptable salt thereof is administered to the subject twice daily at a dose of 30 mg to 60 mg per administration.

40. The compound of Formula (I) or a pharmaceutically acceptable salt thereof of any one of claims 30 to 39, wherein the unit dose of the compound of Formula (I), a stereoisomer thereof, or a pharmaceutically acceptable salt thereof is 2.5 mg to 150 mg.

41. The compound of Formula (I), a stereoisomer thereof, or a pharmaceutically acceptable salt thereof of any one of claims 30 to 40, wherein the unit dose of the compound of Formula (I), a stereoisomer thereof, or a pharmaceutically acceptable salt thereof is 15 mg to 150 mg.

42. The compound of Formula (I), a stereoisomer thereof, or a pharmaceutically acceptable salt thereof of any one of claims 30 to 41, wherein the unit dose of the compound of Formula (I), a stereoisomer thereof or a pharmaceutically acceptable salt thereof is 15 mg, 30 mg, 45 mg, 60 mg, 75 mg, 120 mg, or 150 mg.

43. The compound of Formula (I), a stereoisomer thereof, or a pharmaceutically acceptable salt thereof of any one of claims 30 to 42, wherein the compound of Formula (I), a stereoisomer thereof, or a pharmaceutically acceptable salt thereof is administered to the subject in the form of a tablet or a capsule.

44. The compound of Formula (I), a stereoisomer thereof, or a pharmaceutically acceptable salt thereof of any one of claims 30 to 43, wherein the compound of Formula (I), a stereoisomer thereof, or a pharmaceutically acceptable salt thereof is administered orally to the subject.

45. The compound of Formula (I), a stereoisomer thereof, or a pharmaceutically acceptable salt thereof of any one of claims 30 to 44, wherein the subject is a mammal, preferably a human.

46. The compound of Formula (I) or a pharmaceutically acceptable salt thereof of any one of claims 30 to 45, wherein the subject suffers from a latent tuberculosis infection.

47. The compound of Formula (I) or a pharmaceutically acceptable salt thereof of any one of claims 30 to 46, wherein the subject has a medical history of pulmonary tuberculosis infection, tuberculosis, and/or tuberculous pleurisy.

48. The compound of Formula (I) or a pharmaceutically acceptable salt thereof of any one of claims 30 to 47, wherein titrated dosing is not required.

49. The compound of Formula (I) or a pharmaceutically acceptable salt thereof of any one of claims 30 to 47, wherein the subject suffers from a latent tuberculosis infection, and latent tuberculosis infection is not reactivated after treatment.

50. The compound of Formula (I) or a pharmaceutically acceptable salt thereof of any one of claims 30 to 47, wherein the subject has a medical history of pulmonary tuberculosis infection, tuberculosis, and/or tuberculous pleurisy, and pulmonary tuberculosis infection, tuberculosis, and/or tuberculous pleurisy is not reactivated after treatment.

51. The compound of Formula (I) or a pharmaceutically acceptable salt thereof of any one of claims 30 to 47, wherein the subject has a reduction in Psoriasis Body Surface Area, and/or a reduction in Psoriasis Area and Severity Index, and/or a reduction in Dermatology Life Quality Index.

52. The compound of Formula (I) or a pharmaceutically acceptable salt thereof of any one of claims 30 to 47, wherein the subject's sPGA (Static Physician Global Assessment) is improved, in particular can reach a score of 0 or 1.

53. The compound of Formula (I) or a pharmaceutically acceptable salt thereof of any one of claims 30 to 47, wherein no anxiety is caused or exacerbated in the subject.

54. The compound of Formula (I) or a pharmaceutically acceptable salt thereof of any one of claims 30 to 47, wherein no depressive mood is caused or exacerbated in the subject.

55. The compound of Formula (I) or a pharmaceutically acceptable salt thereof of any one of claims 30 to 47, wherein an improved therapeutic effect in a biological agent-naive subject is also achieved.

56. The compound of Formula (I) or a pharmaceutically acceptable salt thereof of any one of claims 30 to 47, wherein an improved therapeutic effect in a biological agent-experienced subject is also achieved.

57. The compound of Formula (I) or a pharmaceutically acceptable salt thereof of any one of claims 30 to 47, wherein an improved therapeutic effect in a subject who has failed to respond to a biological agent is also achieved.

58. The compound of Formula (I) or a pharmaceutically acceptable salt thereof of any one of claims 30 to 47, wherein immunity of the subject is not reduced.

59. Use of a compound of Formula (I), a stereoisomer thereof or a pharmaceutically acceptable salt thereof in the manufacture of a medicament for treatment of moderate to severe plaque psoriasis in a subject,

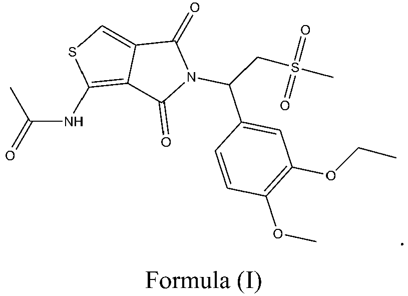

60. The use of claim 59, wherein the compound of Formula (I), a stereoisomer thereof, or a pharmaceutically acceptable salt thereof is administered to the subject at a daily dose of 30 mg to 180 mg.

61. The use of claim 59 or 60, wherein the compound of Formula (I), a stereoisomer thereof, or a pharmaceutically acceptable salt thereof is administered to the subject at a daily dose of 60 mg to 150 mg.

62. The use of claims 59 to 61, wherein the compound of Formula (I), a stereoisomer thereof, or a pharmaceutically acceptable salt thereof is administered to the subject at a daily dose of 60 mg to 120 mg.

63. The use of any one of claims 59 to 62, wherein the compound of Formula (I), a stereoisomer thereof, or a pharmaceutically acceptable salt thereof is administered to the subject at a daily dose of 120 mg.

64. The use of any one of claims 59 to 63, wherein the compound of Formula (I), a stereoisomer thereof, or a pharmaceutically acceptable salt thereof is administered to the subject at a daily dose of 150 mg.

65. The use of any one of claims 59 to 64, wherein the compound of Formula (I), a stereoisomer thereof, or a pharmaceutically acceptable salt thereof is administered to the subject at least once a day.

66. The use of any one of claims 59 to 65, wherein the compound of Formula (I), a stereoisomer thereof, or a pharmaceutically acceptable salt thereof is administered to the subject at least twice a day.

67. The use of any one of claims 59 to 66, wherein the compound of Formula (I), a stereoisomer thereof, or a pharmaceutically acceptable salt thereof is administered to the subject twice daily at a dose of 30 mg to 75 mg per administration.

68. The use of any one of claims 59 to 67, wherein the compound of Formula (I), a stereoisomer thereof, or a pharmaceutically acceptable salt thereof is administered to the subject twice daily at a dose of 30 mg to 60 mg per administration.

69. The use of any one of claims 59 to 68, wherein the unit dose of the compound of Formula (I), a stereoisomer thereof, or a pharmaceutically acceptable salt thereof is 2.5 mg to 150 mg.

70. The use of any one of claims 59 to 69, wherein the unit dose of the compound of Formula (I), a stereoisomer thereof, or a pharmaceutically acceptable salt thereof is 15 mg to 150 mg.

71. The use of any one of claims 59 to 70, wherein the unit dose of the compound of Formula (I), a stereoisomer thereof, or a pharmaceutically acceptable salt thereof is 15 mg, 30 mg, 45 mg, 60 mg, 75 mg, 120 mg, or 150 mg.

72. The use of any one of claims 59 to 71, wherein the compound of Formula (I), a stereoisomer thereof, or a pharmaceutically acceptable salt thereof is administered to the subject in the form of a tablet or a capsule.

73. The use of any one of claims 59 to 72, wherein the compound of Formula (I), a stereoisomer thereof, or a pharmaceutically acceptable salt thereof is administered orally to the subject.

74. The use of any one of claims 59 to 73, wherein the subject is a mammal, preferably a human.

75. The use of any one of claims 59 to 74, wherein the subject suffers from a latent tuberculosis infection.

76. The use of any one of claims 59 to 75, wherein the subject has a medical history of pulmonary tuberculosis infection, tuberculosis, or tuberculous pleurisy.

77. The use of any one of claims 59 to 76, wherein titrated dosing is not required.

78. The use of any one of claims 59 to 76, wherein the subject suffers from a latent tuberculosis infection, and latent tuberculosis infection is not reactivated after treatment.

79. The use of any one of claims 59 to 76, wherein the subject has a medical history of pulmonary tuberculosis infection, tuberculosis, and/or tuberculous pleurisy, and pulmonary tuberculosis infection, tuberculosis, and/or tuberculous pleurisy is not reactivated after treatment.

80. The use of any one of claims 59 to 76, wherein the subject has a reduction in Psoriasis Body Surface Area, and/or a reduction in Psoriasis Area and Severity Index, and/or a reduction in Dermatology Life Quality Index.

81. The use of any one of claims 59 to 76, wherein the subject's sPGA (Static Physician Global Assessment) is improved, in particular can reach a score of 0 or 1.

82. The use of any one of claims 59 to 76, wherein no anxiety is caused or exacerbated in the subject.

83. The use of any one of claims 59 to 76, wherein no depressive mood is caused or exacerbated in the subject.

84. The use of any one of claims 59 to 76, wherein an improved therapeutic effect in a biological agent-naïve subject is also achieved.

85. The use of any one of claims 59 to 76, wherein an improved therapeutic effect in a biological agent-experienced subject is also achieved.

86. The use of any one of claims 59 to 76, wherein an improved therapeutic effect in a subject who has failed to respond to a biological agent is also achieved.

87. The use of any one of claims 59 to 76, wherein immunity of the subject is not reduced.
